# FASCICULE DE BREVET EUROPEEN

(11) **EP 4 025 892 B1**
(45) Date de publication et mention de la délivrance du brevet: **22.10.2025**
(21) Numéro de dépôt: 20764107.7
(22) Date de dépôt: 03.09.2020
(51) Int. Cl.: G01N 15/08, G01N 1/38, G01N 33/483

(54) **DISPOSITIF POUR LA DÉTERMINATION AUTOMATISÉE DU PASSAGE PERCUTANÉ ET DE LA MÉTABOLISATION D'UNE SUBSTANCE**
VORRICHTUNG ZUR AUTOMATISCHEN BESTIMMUNG DER PERKUTANEN PASSAGE UND METABOLISIERUNG EINER SUBSTANZ
DEVICE FOR AUTOMATIC DETERMINATION OF PERCUTANEOUS DIFFUSION AND METABOLIZATION OF A SUBSTANCE

(30) Priorité: 03.09.2019 FR 1909694
(43) Date de publication de la demande: 13.07.2022
(73) Titulaire: Pierre Fabre Dermo-Cosmétique, 81500 Lavaur (FR)
(72) Inventeur: DUPLAN, Hélène, 31320 AUZEVILLE TOLOSAN (FR); JACQUES-JAMIN, Carine, 31170 TOURNEFEUILLE (FR); GENIES, Camille, 31190 AUTERIVE (FR)
(74) Mandataire: Ipside
(86) Numéro de dépôt international: PCT/EP2020/074512
(87) Numéro de publication internationale: WO 2021/043852

(56) Documents cités:
- WO-A1-2004/040006
- WO-A1-2013/057401
- FR-A1- 2 715 471
- M. ALBERTI ET AL: "Multi-chamber microfluidic platform for high-precision skin permeation testing", LAB ON A CHIP, vol. 17, no. 9, 1 January 2017 (2017-01-01), pages 1625 - 1634, XP055669400, ISSN: 1473-0197, DOI: 10.1039/C6LC01574C

## Description

### Domaine technique

L'invention concerne un dispositif pour la détermination automatisée et simultanée du passage percutané et de la métabolisation d'une substance et son impact biologique.

L'invention appartient au domaine des dispositifs *exvivo* ou *in vitro* permettant d'apprécier la pénétration percutanée de substances et leur métabolisation sur des explants maintenus en survie, ainsi que la réponse biologique des explants ou des tissus reconstruits.

La pénétration percutanée est une méthode biopharmaceutique qui évalue l'absorption cutanée d'un principe actif ou d'une substance cosmétique dans une forme galénique.

L'évaluation de la pénétration percutanée est importante notamment pour estimer une toxicité locale.

Le métabolisme, ou biotransformation, représente l'ensemble des réactions biochimiques que subissent les substances endogènes et exogènes, résultant en une diminution de leur lipophilicité et en une augmentation de leur caractère hydrosoluble. Le but ultime du métabolisme est de faciliter l'excrétion de ces substances hors de l'organisme, ce qui est essentiel à sa survie.

L'invention vise à disposer d'un système plus proche des conditions *in vivo,* mettant en œuvre un modèle *ex-vivo* utilisant des explants de peau humaine/animale ou d'un modèle *in vitro* mettant en œuvre des tissus reconstruits (épidermes reconstruits ou modèle reconstruit épiderme/derme), ou des membranes synthétiques.

La connaissance du métabolisme cutané apparait capitale car ce dernier peut être mis à profit pour développer de nouvelles prodrogues mais aussi pour diminuer les risques de toxicité locale de divers xénobiotiques.

### Technique antérieure

La peau est un organe complexe qui a de multiples fonctions.

Elle assure non seulement une fonction de protection, mais participe aussi largement à l'homéostasie thermique et hydrique de l'organisme.

Elle est également impliquée dans la défense immunitaire de l'organisme.

La peau participe au contrôle de l'homéostasie générale de l'organisme en régulant la température ainsi que la perte corporelle en eau et en électrolytes.

La peau est une barrière physique à l'entrée de xénobiotiques dans l'organisme. Ce rôle de barrière est principalement joué par le *stratum corneum.* Cependant, cette barrière n'est pas totale et la peau reste perméable à certaines molécules. Le *stratum corneum* joue un rôle capital dans ces fonctions et peut-être considéré comme un réservoir à partir duquel la substance stockée peut diffuser vers les couches les plus profondes de la peau.

Anatomiquement, deux voies distinctes s'offrent pour la pénétration des substances : d'une part, la voie transépidermique et d'autre part, la voie transfolliculaire.

Pour la voie transépidermique, la diffusion de la molécule s'effectue soit à travers les cellules cornées, essentiellement constituées de protéines hydrophiles, soit à travers les espaces intercellulaires de la couche cornée, constitués de lipides.

Du fait de son caractère amphiphile, le domaine intercellulaire hydrolipidique constitue un canal de diffusion préférentiel pour les substances lipo- et hydro-solubles.

Les substances lipophiles diffusent à travers les zones hydrophobes des bicouches lipidiques intercellulaires. Les composés plus hydrophiles migrent, d'une part, à travers les zones hydrophiles des bicouches lipidiques intercellulaires et d'autre part, en utilisant la voie intracellulaire.

Les substances lipophiles peuvent également emprunter la voie transfolliculaire via les follicules pilosébacés et/ou les glandes sudoripares. Cependant, cette voie reste minoritaire.

La pénétration ne s'effectue pas uniquement par l'une des voies. Les deux participent au phénomène, et la pénétration globale est la résultante d'un passage transépidermique et d'un passage par les annexes cutanées.

Même si le passage percutané peut-être faible en fonction des composés d'intérêt, celui-ci peut être déterminant pour certaines substances.

En effet, le *stratum corneum* limite l'absorption percutanée de xénobiotiques grâce à son effet barrière.

La peau est en effet capable de métaboliser les composés avant leur passage dans la circulation sanguine et cette biotransformation est susceptible d'influencer la diffusion d'un composé à travers la peau.

L'épiderme possède des enzymes fonctionnelles qui vont biotransformer les xénobiotiques ayant traversés le *stratum corneum,* en particulier, les composés lipophiles en molécules plus hydrophiles qui vont diffuser plus facilement à travers l'épiderme et le derme.

Le métabolisme peut ainsi avoir une influence sur la diffusion percutanée de composés très peu solubles ou lipophiles et modifier les propriétés des molécules et leur impact sur le tissu.

Pour l'évaluation du passage percutané, Franz et Bronaugh ont développé la première cellule de mesure de diffusion percutanée en 1975.

Il existe deux types de cellules : les cellules statiques et les cellules à flux dites dynamiques, où le liquide est renouvelé de manière continue.

Ces cellules comprennent deux compartiments : le compartiment donneur séparé du compartiment récepteur par une membrane, ladite membrane étant un explant cutané lorsque la diffusion percutanée est étudiée.

Le compartiment donneur reçoit la formulation contenant la substance à tester et des prélèvements successifs dans le compartiment récepteur permettent d'établir, au cours du temps, le profil cinétique de passage.

En fin d'expérience, la quantité d'actif qui n'a pas pénétré dans la peau est mesurée ainsi que la distribution dans les différentes couches cutanées ( *stratum corneum,* épiderme, derme) et la quantité présente dans le compartiment récepteur.

Ces données permettent d'établir un bilan massique, un profil de distribution et de calculer un certain nombre de paramètres, dont le coefficient de perméabilité, de partage ou de diffusion.

Cependant, l'utilisation des dispositifs de l'art antérieur présente des insuffisances conduisant à des incertitudes sur les résultats obtenus et ne permettent pas l'étude conjointe du passage percutané et du métabolisme ainsi que la réponse biologique des explants ou des tissus reconstruits.

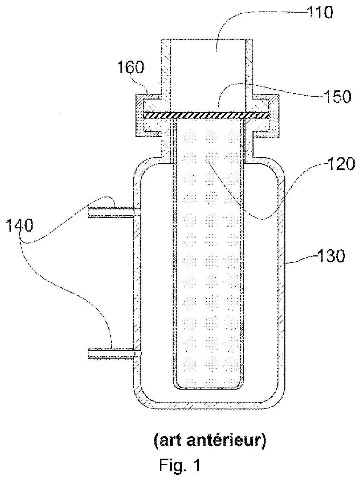

La figure 1, relative à l'art antérieur, montre une représentation de principe d'une cellule de diffusion dite de Franz, utilisée pour l'analyse *in vitro* ou *ex vivo* de la diffusion d'une substance à travers une membrane et notamment à travers un explant de peau.

Selon cet exemple, le dispositif de l'art antérieur comprend un compartiment donneur (110) et un compartiment récepteur (120).

Le compartiment récepteur est contenu dans un flacon à double paroi (130) et des moyens (140) permettent de réaliser une circulation fluide entre lesdites parois du flacon, notamment afin de maintenir l'ensemble à une température prédéterminée.

La substance à tester est déposée dans le compartiment donneur (110). Celle-ci ne peut passer dans le compartiment récepteur (120) qu'en traversant la membrane (150).

Ladite membrane (150) est en effet prise en sandwich entre l'extrémité inférieure de l'enveloppe du compartiment donneur et l'extrémité supérieure du flacon (130) à double paroi comprenant le compartiment récepteur.

Des moyens de serrage (160) permettent d'assembler les trois pièces (110, 130, 150) de manière étanche.

D'autres moyens (non représentés) permettent de prélever des échantillons dans le compartiment récepteur (120) au moyen d'une seringue sans avoir à ouvrir le dispositif.

Ainsi, selon ce dispositif de l'art antérieur, l'étanchéité, à la fois entre le compartiment donneur (110) et le compartiment récepteur (120), mais aussi entre le fluide circulant entre les parois du flacon (130) et ces compartiments, est assurée par la membrane (150) elle-même et le serrage sur cette membrane.

Or, lorsque la membrane est un explant de peau, la présence de ce serrage ne permet pas de maintenir ledit explant en survie.

En effet, le serrage induit une nécrose du tissu qui se propage rapidement et altère la fiabilité du résultat lors de l'utilisation d'explants maintenus en survie. Ainsi, ce système n'est pas adapté pour étudier le métabolisme d'une substance par des modèles de peau en survie, ainsi que la réponse biologique des explants ou des tissus reconstruits.

Ce serrage est toutefois nécessaire et doit être suffisant pour assurer une étanchéité irréprochable du système sous réserve de doser une proportion d'actif n'ayant pas réellement diffusé dans l'explant.

Les conditions de maintien en survie de l'explant (température, humidité, taux de CO₂) nécessitent de placer l'expérimentation dans un milieu adéquat.

Les évaluations de métabolisme cutané sont classiquement réalisées dans un incubateur en présence de CO2, d'un milieu de culture contenant des nutriments pour les modèles de peau et d'une atmosphère saturée en eau permettant de maintenir la peau en survie.

Ledit incubateur est un système statique composé d'une plaque de culture avec des puits contenant le milieu de culture dans lequel se trouve un insert composé d'une membrane. Sur l'insert est placé le tissu reconstruit ou l'explant de peau.

La substance est déposée à la surface du tissu cutané.

Le métabolisme est évalué par la quantification de la substance testée et de ses métabolites dans les différents compartiments du modèle (surface, *stratum corneum,* peau et milieu récepteur).

Le tissu cutané recouvre la surface de l'insert mais l'absence de système de serrage de la peau contre l'insert ne permet pas d'assurer une étanchéité et peut entrainer une fuite de la substance testée dans le puits où se trouve le milieu récepteur.

Comme évoqué ci-dessus, la possible fuite de l'actif dans le milieu sans passage par l'explant est une problématique systématiquement reprochée à ce type d'essai lors de l'utilisation de plaques de culture avec inserts ne permettant pas de créer une étanchéité. Ce système n'est donc pas adéquat pour l'évaluation du passage transcutané.

Les conditions d'hygrométrie et de température bien qu'assurant la viabilité du modèle de peau, peuvent modifier les propriétés d'absorption cutanée et ne miment pas des conditions *in vivo* (hygrométrie, température, système statique).

La température ainsi que l'hydrométrie peuvent jouer sur l'organisation lipidique, l'hydratation du *stratum corneum,* la viabilité des enzymes cutanées mais également sur les paramètres physicochimiques des substances à tester.

Il est à noter que ce système statique s'éloigne de la situation *in vivo* où on retrouve la circulation sanguine. L'absence de flux, peut conduire à une métabolisation plus importante des composés par recaptage dans le milieu.

Le document « Multi-Chamber microfluidic platform for high-precision skin permeation testing » M. Albert et al., Lab On a Chip, vol. 17, n°9, 2017-01-01, p 1625-1634 décrit une cellule comprenant un compartiment donneur et un compartiment récepteur pour l'étude du passage percutané d'une substance, laquelle cellule comprend un dispositif microfluidique pour la circulation d'un fluide au contact de l'explant dans le compartiment récepteur.

Ce dispositif, s'il apporte des perfectionnements à la cellule de Frantz décrite plus haut, réalise toujours l'étanchéité entre les deux compartiments par l'intermédiaire d'un serrage sur l'explant et par conséquent présente les mêmes risques de nécrose et les mêmes difficultés de maintien en vie dudit explant.

### Exposé de l'invention

L'invention vise à résoudre les inconvénients de l'art antérieur et concerne à cette fin une cellule de diffusion permettant de maintenir un explant cutané en survie tout en assurant une étanchéité parfaite et se rapprochant des conditions *in vivo.*
A cette fin, la cellule objet de l'invention est telle que définie dans la revendication 1 et comprend :
- un explant de peau humaine/animale ou épiderme/peau reconstruit ;
- un puits comprenant une cavité constituant un compartiment récepteur ;
- un chapeau comprenant un compartiment donneur ;
- des moyens de connexion du puits et du chapeau ;
dans lequel, l'explant est maintenu entre le compartiment donneur et le compartiment récepteur entre deux joints, l'un des joints étant lié au puits dans un logement pratiqué dans ledit puits et l'autre au chapeau dans un logement pratiqué dans ledit chapeau, et qu'il comprend des moyens pour régler la pression exercée par les joints sur l'explant.

Ainsi, l'utilisation de moyens spécifiques permet de définir une pression maximale admissible sur les joints, apte à assurer l'étanchéité mais suffisamment faible pour ne pas entraîner de nécrose de l'explant et maintenir ce dernier en survie.

L'invention est avantageusement mise en œuvre selon les modes de réalisation exposés ci-après, lesquels sont à considérés individuellement ou selon toute combinaison techniquement opérante.

Selon un mode de réalisation, les moyens de réglage de la pression exercée par les joints comprennent des moyens d'interface répartis entre le chapeau et le puits et qui définissent, lorsqu'ils sont en contact, une surface de pose du chapeau sur le puits, et des moyens de réglage permettant de modifier la distance entre la surface de pose et le logement de joint pratiqué dans le chapeau ou le logement de joint pratiqué dans le puits.

Ces moyens permettent de régler la pression exercée par les joints sur l'explant en fonction de l'épaisseur de celui-ci.

Selon un mode de réalisation les moyens d'interface du chapeau et du puits sont maintenus l'un contre l'autre par une force d'attraction magnétique.

Cette disposition sécurise l'assemblage tout en conservant les possibilités de réglage.

Avantageusement, la cellule objet de l'invention comprend une membrane perméable ou semi-perméable maintenue contre l'explant et apte à maintenir l'explant dans une configuration sensiblement plane.

Ainsi, la cellule objet de l'invention est utilisable tant verticalement qu'horizontalement sans que l'explant ne ploie.

Dans une configuration, le compartiment du chapeau est le compartiment donneur et la cavité du puits est le compartiment récepteur.

Selon un mode de réalisation, la cellule objet de l'invention comprend des conduits pour le passage d'un fluide dans la cavité du puits, lesquels conduits débouchent dans la cavité du puits, et des moyens, connectés auxdits conduits pour le contrôle du débit du fluide dans ladite cavité

Ainsi, l'explant peut être maintenu en température et en survie par la circulation dudit fluide lui apportant les nutriments nécessaires.

Avantageusement, les moyens de contrôle du débit du fluide dans la cavité comprennent une pompe péristaltique agissant en refoulement dans l'un des conduits et une pompe péristaltique agissant en aspiration dans l'autre conduit.

Cette disposition permet d'assurer le passage d'un fluide à un débit très faible et à faible pression, ainsi que d'effectuer une collecte du fluide à intervalle de temps donné. L'utilisation de pompes péristaltiques évite la formation de bulles d'air dans l'écoulement.

Avantageusement, la cavité du puits est délimitée à une de ses extrémités par l'explant et à son extrémité opposée par une plaque de fermeture, ladite plaque de fermeture étant constituée d'une matière transparente de sorte à permettre l'observation visuelle du passage du fluide dans ladite cavité.

Cette disposition permet de vérifier l'absence de bulles dans la cavité du puits.

L'invention concerne également, un dispositif comprenant une pluralité de cellules selon l'un quelconque des modes de réalisation précédents, et comprenant :
- une enceinte et des moyens de régulation aptes à maintenir la pluralité de cellules dans des conditions de température et d'environnement contrôlées ;
- des moyens pour le passage d'un fluide dans les cavités de puits de chaque cellule ;
- des moyens pour la collecte du fluide passé dans les cavités de puits de chaque cellule.

Avantageusement, les cellules du dispositif objet de l'invention sont montées dans un rack, ledit rack comprenant une surface miroir permettant de refléter l'image observer dans chaque cellule à travers sa plaque de fermeture.

Cette disposition facilite le contrôle de l'absence de bulle dans le fluide lors de son passage dans les cavités de puits lorsque les cellules sont montées en rack.

Selon un mode de réalisation du dispositif objet de l'invention, les moyens de passage d'un fluide comprennent un réservoir de fluide, une première pompe aspirant le fluide dans ledit réservoir et l'injectant dans la cavité de puits de chaque cellule par l'un des conduits desdites cellules et une deuxième pompe aspirant le fluide dans la cavité de puits de chaque cellule par l'autre conduit desdites cellules et le refoulant dans les moyens de collecte.

Selon un mode de réalisation, les moyens de collecte comprennent une station d'entrée comprenant des moyens pour connecter les conduits au refoulement de la deuxième pompe, une station de prélèvement comprenant une pluralité de becs de remplissage chacun d'entre eux en connexion hydraulique avec un des conduits de refoulement de la deuxième pompe, un rack apte à contenir une pluralité de flacons et à positionner lesdits flacons en vis-à-vis de chaque bec de remplissage, des moyens de déplacement relatif dudit rack par rapport à la station de prélèvement de sorte à changer les flacons placé en vis-à-vis de chaque bec de remplissage.

Ainsi, en programmant le déplacement du rack comprenant les flacons par rapport à la station de prélèvement, ce dispositif permet de réaliser des prélèvements à intervalle de temps défini, du fluide ayant traversé la cavité de puits de chaque cellule.

Avantageusement, les moyens de collecte comprennent des moyens pour réfrigérer les flacons compris dans le rack. Ces moyens assurent une meilleure conservation des prélèvements.

### Description sommaire des dessins

L'invention est exposée ci-après selon ses modes de réalisations et variantes préférés, nullement limitatifs et en référence des figures 1 à 13 dans lesquelles :
la figure 1 relative à l'art antérieur montre, selon une vue en coupe, un exemple de réalisation d'une cellule de diffusion selon l'art antérieur ;
la figure 2 représente selon une vue en perspective et en éclaté, un exemple de réalisation d'une cellule de diffusion selon l'invention ;
la figure 3 montre selon une vue en coupe AA, définie figure 2 un exemple de réalisation d'une cellule selon l'invention ;
la figure 4 représente schématiquement une vue de dessus un exemple de réalisation d'un dispositif automatisé mettant en œuvre une pluralité de cellules selon l'invention ;
la figure 5 montre selon une vue en perspective simplifié, un exemple de réalisation du dispositif automatisé de récupération du dispositif de la figure 4 ;
la figure 6 illustre selon une vue en perspective, un exemple de montage en rack d'une pluralité de cellules dans le dispositif de la figure 4 ;
la figure 7 compare la pénétration cutanée du résorcinol et de ses métabolites 24 h après application topique (% de la dose appliquée) mesurée au moyen du dispositif objet de l'invention et selon un essai de référence sur des inserts en plaque de culture dans un incubateur classique ;
la figure 8 montre un comparatif des résultats obtenus avec le dispositif objet de l'invention sur le métabolisme cutané du résorcinol 24 h après application topique (nanomoles dans milieu + peau), en comparaison d'un essai de référence sur des inserts en plaque de culture dans un incubateur classique ;
la figure 9 montre le dispositif automatisé de récupération de la figure 5 selon une vue de droite en référence à cette figure, dans un mode de réalisation comprenant des moyens de réfrigération.
la figure 10 montre le résultat d'un essai de pénétration cutanée du propylparaben et de ses métabolites 24 h après une application topique (% de la dose appliquée), réalisé au moyen du dispositif objet de l'invention et en comparaison d'un essai de référence sur des inserts en plaque de culture dans un incubateur classique ;
la figure 11 montre un résultat d'essai portant sur le métabolisme cutané du propylparaben 24 h après application topique (nanomoles dans milieu + peau), mis en œuvre au moyen du dispositif objet de l'invention et en comparaison d'un essai de référence sur des inserts en plaque de culture dans un incubateur classique ;
la figure 12 est un exemple de résultat d'un essai mis en œuvre au moyen du dispositif objet de l'invention, portant sur la pénétration cutanée de la testostérone et de ses métabolites 24 h après application topique (% de la dose appliquée) et en comparaison d'un essai de référence sur des inserts en plaque de culture dans un incubateur classique ;
et la figure 13 représente un exemple de résultat d'essai mis en œuvre au moyen du dispositif objet de l'invention et portant sur le métabolisme cutané de la testostérone 24 h après application topique (nanomoles dans milieu + peau) et en comparaison d'un essai de référence sur des inserts en plaque de culture dans un incubateur classique.

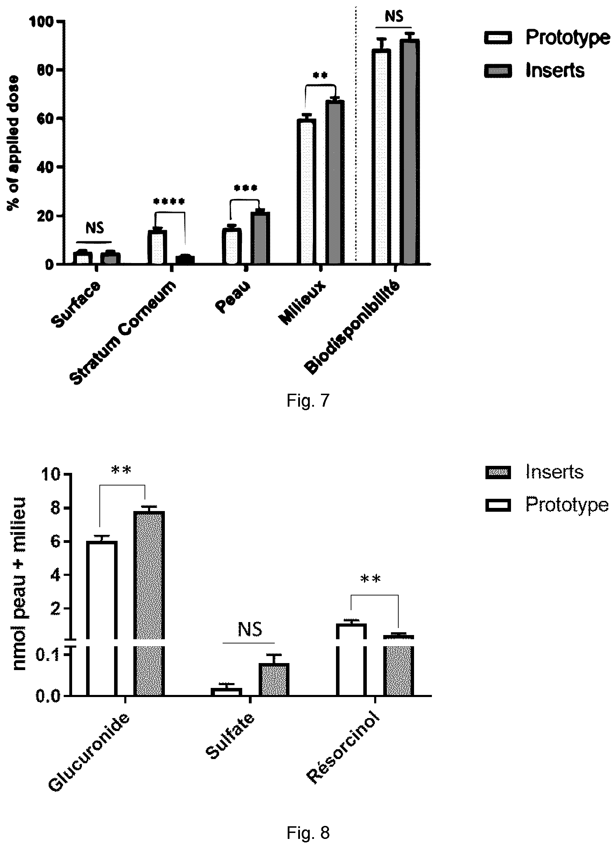

Dans les figures 7, 8, 10, 11, 12 et 13 les résultats référencés « prototype » correspondent à des résultats obtenus avec un exemple de réalisation du dispositif objet de l'invention.

### Manière(s) de réaliser l'invention

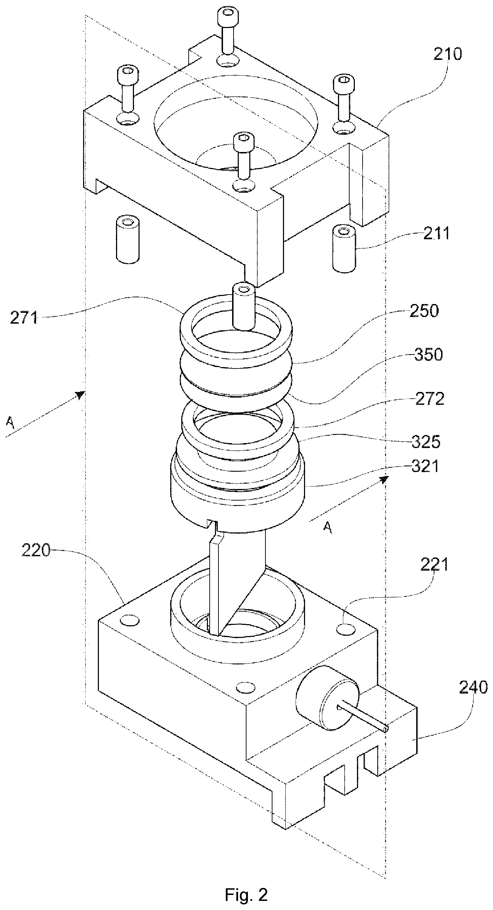

Figure 2 selon un exemple de réalisation la cellule objet de l'invention est constituée par l'assemblage de deux pièces principales (210, 220), l'une étant dénommée puits (220) et l'autre chapeau (210).

Ces deux pièces comprennent chacune un alésage, définissant une cavité, lesquels alésages sont susceptibles de contenir un produit.

L'une des pièces, ici le puits selon ce mode de réalisation, comporte avantageusement une forme (240) pour le montage de ladite cellule en rack.

Lorsqu'ils sont assemblés entre eux de sorte à constituer la cellule, le chapeau (210), selon cet exemple de réalisation, repose sur 4 plots (221) réglables, qui entrent en contact avec les extrémités de 4 aimants (211) montés dans le chapeau (210).

A titre d'exemple non limitatif, les plots (221) réglables sont en pratique les extrémités de vis, vissées dans le puits et manœuvrables par leurs extrémités opposées.

L'explant (250) est maintenu entre le chapeau (210) et le puits (220) entre deux joints (271, 272) élastiques souples.

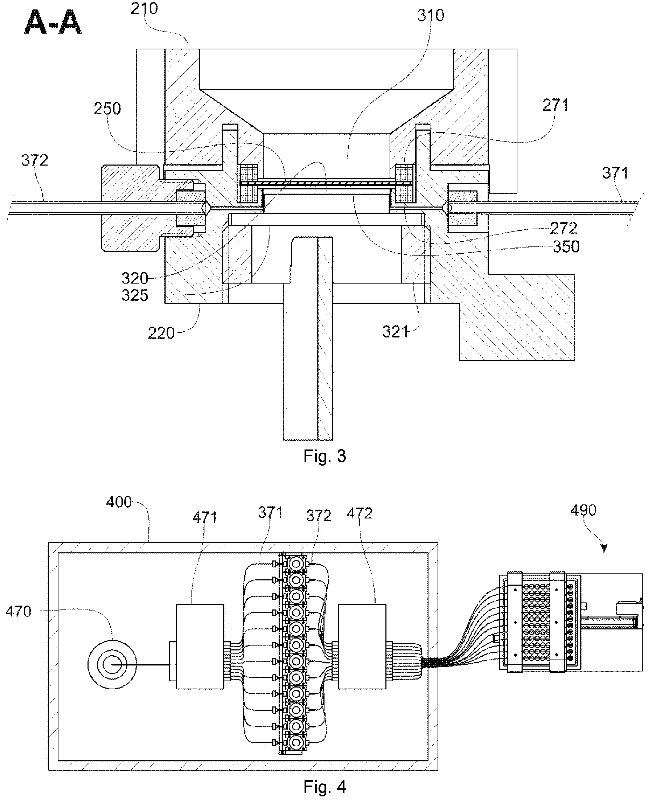

La figure 3 montre l'ensemble de la figure 2 assemblé.

Figure 2 et figure 3, la cellule objet de l'invention est représentée dans un mode de réalisation où la substance est déposée sur l'explant (350) dans le compartiment donneur (310) délimité par les parois du chapeau (210) et où le compartiment récepteur (320) et réalisé par une cavité du puits (220).

La cavité (320) du puits est délimitée par les parois de l'alésage du puits et une pièce de fermeture (325) en verre ou dans une matière transparente, laquelle est serrée contre un épaulement au moyen d'un bouchon fileté (321) ce serrage assurant l'étanchéité de ladite cavité, fermée à son autre extrémité axiale par l'explant (250).

Selon ce mode de réalisation, la cavité (320) du puits est de plus isolée de l'explant par une membrane (350) perméable ou semi-perméable appliquée contre l'explant (250).

Ladite membrane maintient notamment l'explant dans une configuration sensiblement plane évitant que celui-ci ne s'incurve et ne modifie les conditions de débit dans la cavité.

Figure 3, les joints (271, 272) sont centrés sur des portions cylindriques en saillie sur les faces en vis-à-vis du chapeau et du puits et prennent appui sur des surfaces, ici horizontales, respectivement du chapeau et du puits.

Figure 2, si l'ensemble est en position verticale comme le représente cette figure, le poids du chapeau est repris par les plots (221) du puits. Pour une épaisseur d'explant donnée, et une souplesse donnée des joints, la pression sur lesdits joints (271, 272) dépend de la distance entre la surface d'appui des aimants (211) sur les plots (221) et les surfaces d'appuis des joints dans le chapeau et dans le puits.

Cette distance est réglée en ajustant la hauteur des plots (221) et la position axiale des aimants.

En effectuant ce réglage, il est ainsi possible d'appliquer une pression juste suffisante pour assurer l'étanchéité sans risquer de dégrader le tissu constituant l'explant.

En revenant figure 3, selon ce mode de réalisation, des conduits (371, 372) débouchant dans le compartiment récepteur (320) permettent de faire circuler un fluide dans ce compartiment afin de maintenir l'explant en survie

Les aimants (211) sont sélectionnés de sorte que la force d'attraction qu'il exercent soit suffisante et que la pression dans la cavité (320) du puits, en pratique très faible, appliquée lors de la circulation du fluide ne risque pas de casser l'étanchéité.

Ainsi, selon ce mode de réalisation, la substance objet de l'essai est appliquée sur l'explant dans le compartiment donneur (310) constitué dans le chapeau, et diffuse à travers l'explant (250) vers le compartiment récepteur (320) constitué dans le puits, compartiment récepteur dans lequel elle va se mêler au fluide.

L'homme du métier comprend que le système peut être inversé et que la cavité (320) du puits peut agir comme compartiment donneur et la cavité (310) du chapeau agir comme compartiment récepteur, au prix de modification mineures, permettant ainsi, par exemple, l'étudier la diffusion de substances incorporées dans un flux à travers l'explant.

Les conduits (371, 372) sont chacun connectés à une pompe péristaltique de sorte à assurer le passage du fluide dans la cavité (320) sans création de bulle d'air dans ledit fluide.

La pièce de fermeture en verre (325) permet d'observer le passage du fluide et de vérifier l'absence de bulle dans celui-ci.

En pratique, deux pompes péristaltiques agissant l'une en refoulement et l'autre en aspiration, sont connectée aux conduits, par exemple la pompe agissant en refoulement est connectée au conduit (371) d'injection à droite de la figure 3 et la pompe agissant en aspiration est connectée au conduit (372) d'extraction à gauche de la figure 3.

Ainsi, le fluide est injecté dans la cavité (320) par l'un des conduits (372), cavité dans laquelle la substance en test diffuse dans ledit fluide à travers l'explant (250), et le fluide mêlé à la quantité de substance ayant diffusé à travers l'explant est récupérable à l'extrémité de l'autre conduit.

Ainsi, ce système permet un passage régulier de fluide dans la cavité (320) du puits, et non une circulation, et d'effectuer par prélèvement régulier, une analyse de la quantité de substance ayant diffusée dans ledit fluide, à travers l'explant, au cours du temps.

En pratique, le volume de fluide présent dans la cavité du puits est renouvelé selon une périodicité de l'ordre de 2 heures, sans que cette valeur ne soit limitative.

Figure 4, selon un exemple de réalisation du dispositif objet de l'invention, celui-ci comprend au moins une cellule et en pratique une pluralité de cellules, ici 12 au maximum, telles que décrites ci-avant.

Les cellules sont placées dans une enceinte (400) dont la température et les conditions environnementale sont contrôlées.

A titre d'exemple la température à l'intérieur de l'enceinte est maintenue à une température 37°C pour une hygrométrie de 60 %. Le taux de CO₂ dans l'enceinte est également contrôlé et maintenu à une valeur définie.

Selon des exemples de réalisation, l'environnement dans l'enceinte est également contrôlé en termes de propreté et de stérilité ainsi que de pression.

Ces conditions environnementales contrôlées permettent un meilleur maintien en survie des explants et, notamment par le contrôle de l'hygrométrie, de conserver la perméabilité des explants en évitant que ceux-ci ne se saturent en humidité.

Le dispositif comprend un réservoir (470) contenant le fluide dont le passage est assuré dans la cavité de puits des cellules par une première (471) ou une pluralité de premières pompes péristaltiques en parallèle, aspirant le fluide dans le réservoir (470) et refoulant dans les conduits (371) d'injection dudit fluide dans chaque cellule.

Chaque cellule est alimentée individuellement en fluide dans sa cavité de puits.

Le réservoir est, selon cet exemple de réalisation, placé dans l'enceinte (400) de sorte à se trouver également à température et conditions environnementales contrôlées. Selon un autre exemple de réalisation (non représenté), ledit réservoir est placé à l'extérieur de l'enceinte et comprend ses propres moyens de régulation, notamment en température.

Une seconde pompe péristaltique (472) ou une pluralité de secondes pompes péristaltiques en parallèle, aspirent par l'intermédiaire des conduits d'extraction (372) de chaque cellule, ledit fluide et refoulent ledit fluide vers un système (490) automatisé de collecte d'échantillons ou de prélèvement.

Ce dispositif comprenant deux pompes ou deux séries de pompes péristaltiques (471, 471), permet de réaliser un passage à débit continu du fluide au contact de l'explant dans chacune des cellules, ou un renouvellement intermittent, à fréquence régulière ou non dudit fluide dans chaque cavité de chaque cellule du dispositif.

Ainsi, chaque cellule reçoit en début d'essai une dose de substance dont l'étude de la pénétration/métabolisation percutanée est visée, dans le compartiment donneur constitué par la cavité du chapeau, la substance venant en contact avec l'explant.

Selon des exemples de mise en œuvre, la même substance est déposée dans chaque cellule, chacune comprenant le même type d'explant, ou, plusieurs substance et/ou plusieurs type d'explants sont utilisés et diffèrent d'une cellule à l'autre.

Dans chaque cellule, la substance diffuse à travers l'explant et passe dans le fluide de la cavité de puits.

Ledit fluide est extrait de chaque cellule, et collecté dans des flacons individuels par le système de collecte (490) à intervalles réguliers.

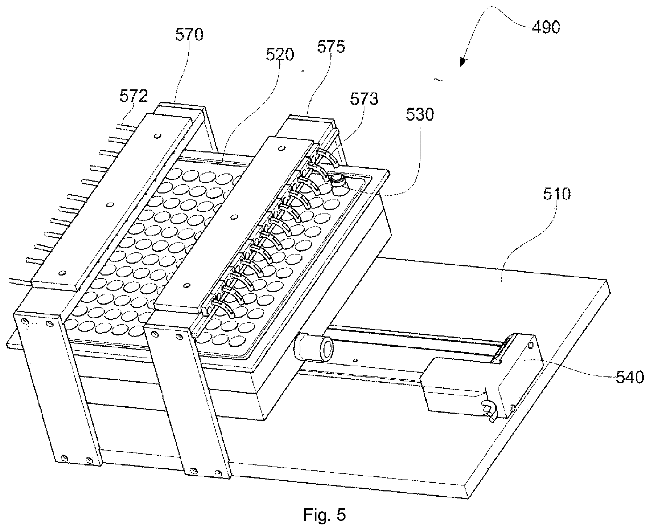

Figure 5, le système automatisé de collecte d'échantillons est représenté de manière simplifiée pour en faciliter la lecture, notamment, tous les flacons de collecte ne sont pas représentés et seules les parties terminales des conduits sont représentées.

Le système comprend une base (510) fixe et un rack (520) apte à contenir une pluralité de flacons (530).

Selon cet exemple de réalisation, le dispositif comprend 12 cellules, aussi le rack (520) comprend N colonnes de 12 emplacements pour Nx12 flacons.

Le rack (520) est déplaçable relativement à la base fixe (510) par des moyens de motorisation.

Selon des exemples de réalisation non limitatifs, lesdits moyens de motorisation comprennent un moteur pas à pas associé à un système pignon crémaillère ou à un système à douille et vis-à-bille ou encore les moyens de motorisation comprennent un moteur linéaire.

Les conduits (572) connectés à la sortie (refoulement) de la seconde pompe péristaltique (472, figure 4) sont connectés à une station d'entrée (570), et sont en communication hydraulique (non représentée) de manière individuelle avec un bec de remplissage (573) situé sur une station de prélèvement (575).

La station de prélèvement est fixe par rapport à la base (510).

Chaque bec de remplissage (573) de la station de prélèvement est situé en face d'un emplacement pour un flacon (530), de sorte que, selon cet exemple de réalisation, la station de prélèvement comprend 12 becs de remplissage (530), la station d'entrée comprenant 12 connexions pour les conduits (572) issues de la seconde pompe péristaltique du dispositif.

Ainsi, ce système permet de réaliser des prélèvements sur un maximum de 12 cellules.

Lorsque moins de cellules, par exemple 3, 6, 10 ou 9, sont utilisés, seules une partie des emplacements du rack sont utilisées et seul les conduits (572) correspondants auxdites cellules sont connectés.

Les moyens de motorisation (540) permettent de déplacer le rack (520) comprenant les flacons (530) de sorte à déplacer chaque colonne dudit rack en face des becs de remplissage de la station de prélèvement, et ainsi d'effectuer des prélèvements selon une périodicité ou un planning défini.

Le contenu de chaque flacon est ensuite analysé selon toute technique appropriée.

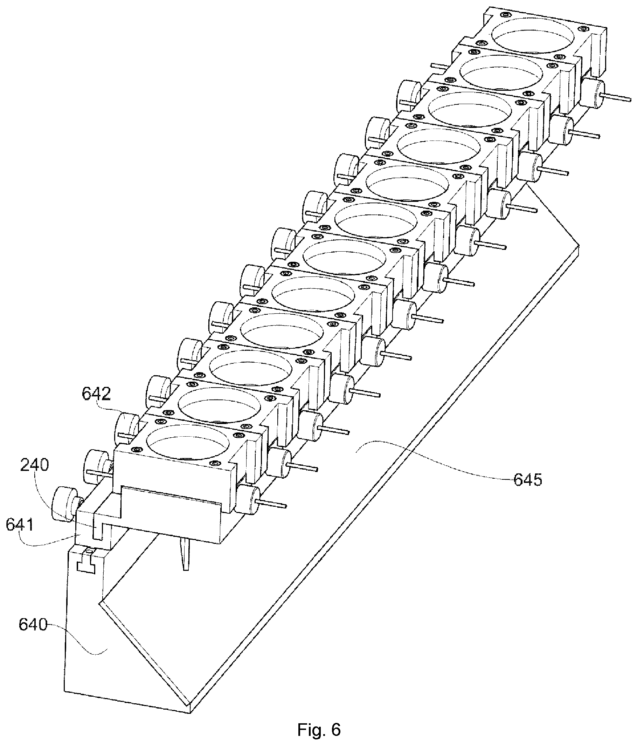

Figure 6, pour la mise en œuvre du dispositif objet de l'invention, le nombre de cellules utilisées pour l'expérimentation, ici un maximum de 12, est monté dans un rack (640).

A cette fin, ledit rack comprend une forme complémentaire, une rainure (641) selon cet exemple de réalisation, apte à recevoir la forme (240) de la cellule utilisée à cette fin et des moyens de serrage (642) pour maintenir lesdites cellules en position.

Selon cet exemple de réalisation, la surface (645) du rack en vis-à-vis de des plaques de fermetures (325, figure 3) des cellules est un miroir.

Ainsi, ledit miroir permet d'observer facilement l'écoulement du fluide dans la cavité de puits des cellules afin de, notamment, de vérifier l'absence de bulles dans cet écoulement.

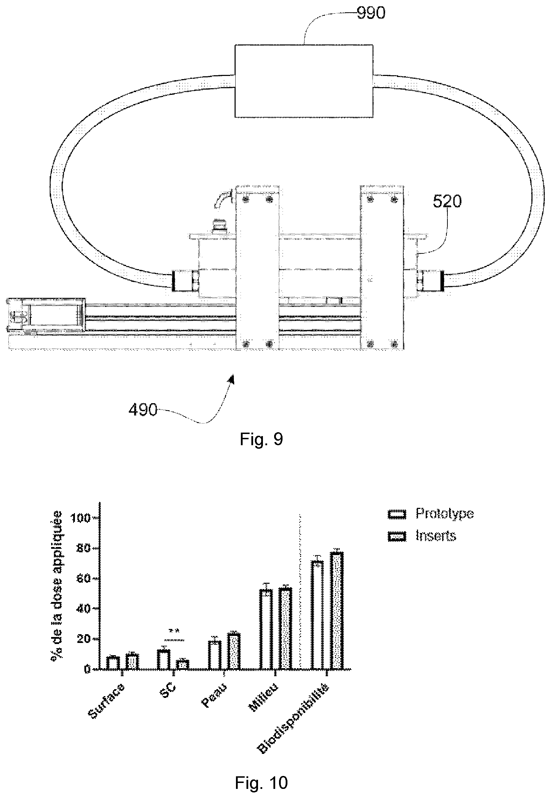

Figure 9, le système (490) automatisé de collecte de prélèvements comprend avantageusement des moyens pour contrôler la température des flacons de prélèvements.

Selon un exemple de réalisation, ces moyens comprennent un dispositif (990) pour la circulation d'un fluide caloporteur thermostaté dans la partie inférieure du rack (520) supportant les flacons, lequel rack se présente alors comme un bac étanche.

Ains, les flacons de prélèvement sont au contact du fluide caloporteur, ce qui permet de les maintenir à une température appropriée, par exemple 4°C, de sorte que les prélèvements ainsi réfrigérés se conservent sans dégradation.

Les exemples suivants illustrent, sur des exemples, les avantages de l'invention sans en limiter la portée.

### Exemple 1 : Coupes histologiques de peau

Les inventeurs ont comparé des coupes histologiques de peau utilisées en explant soit dans le dispositif selon l'invention soit dans des plaques de culture avec inserts afin d'évaluer l'intégrité du tissu et le maintien en survie.

L'étude a été réalisée sur 4 donneurs pour les cellules du dispositif selon l'invention, et en parallèle pour les inserts en plaque de culture dans un incubateur classique qui va servir de comparateur.

La durée de l'étude est de 24 heures, il n'y a aucun dépôt de produit. L'étude est réalisée sur des explants de peau d'oreille de porc maintenue en survie. La peau d'oreille de porc est un modèle cutané validé par les recommandations relatives à l'étude du passage percutané, comme modèle alternatif à la peau humaine (OECD 428 and SCCS). Le milieu complet comprend 100 mL de DMEM (Dulbecco's Modified Eagle Medium, sans rouge de phénol, 4 mL de L-glutamine (200 mM), 2 mL de streptomycine/pénicilline (100µg/mL),1 mL de fongizone (2.5 µg/mL) et 100 µL de gentamycine (50 µg/mL).

Trois disques de 8mm de diamètre sont découpés par emporte-pièce dans chaque explant de peau. Les disques sont inclus en paraffine en vue de leur analyse en microscopie optique après coloration hémalum/éosine.

Les inventeurs n'observent pas de différences notables sur les coupes histologiques des explants maintenus en survie sur inserts en plaque de culture et les explants maintenus en survie sur les cellules du dispositif selon l'invention.

Le *stratum corneum* est bien fixé et la couche basale est normale, les tissus supportent bien l'enceinte du dispositif selon l'invention ainsi que le serrage qui permet de créer une étanchéité entre les deux compartiments donneur et récepteur dans le système dynamique.

### Exemple 2 : test de viabilité et de cytotoxicité

Les inventeurs ont conduit des tests pour estimer la viabilité des explants de peau dans le dispositif selon l'invention, des études sont menées en parallèles avec des inserts en plaque de culture dans un incubateur classique permettront de comparer ces tests.

Les études ont été menées sur 4 donneurs en triplicat (n=3) par donneur et pour chaque dispositif. Le test MTT est utilisé comme méthode rapide de numération des cellules vivantes via la conversion du sel de tétrazolium MTT (bromure de 3-(4,5-dimethylthizol-2-yl)-2,5-diphenyl tetrazolium) par la succinate déshydrogénase mitochondriale des cellules vivantes actives, en formazan.

Le formazan forme un précipité dans la mitochondrie de couleur violette. La quantité de précipité formée est proportionnelle à la quantité de cellules vivantes.

Le test LDH est utilisé comme marqueur des lésions des tissus via la libération dans le milieu de culture de la lactate déshydrogénase par le tissu évalué. La LDH étant une enzyme intracellulaire, elle est relarguée dans le milieu en cas de lyse cellulaire ou d'une altération tissulaire.

Trois séries avec 4 donneurs ont été réalisées soit avec le dispositif selon l'invention soit avec les inserts en plaque de culture dans un incubateur classique.

La méthodologie de mise en culture est la même que celle décrite dans l'exemple 1.

Après 24h, les études sont stoppées et les explants de peau sont placés sur des inserts de culture pour le dosage MTT. La mesure est effectuée par densité optique à 570nm en microplaques 96 puits.

Les milieux ont été conservés pour le dosage de la LDH (Lactate Déshydrogénase). La lecture est effectuée par densité optique à 490nm et 620nm.

### Résultats dosage MTT :

Les résultats sont résumés dans le tableau 1 ci-dessous

| | DO/cm²Cellules statiques | DO/cm² Cellules prototypes | % viabilité (Invention / Statique) |
|---|---|---|---|
| Moyenne | 0,0398 | 0,0481 | 120,86 |
| SEM | 0,0086 | 0,0063 | / |

DO : Densité optique ; % viabilité est calculé en tenant compte des cellules du dispositif selon l'invention par rapport aux inserts en plaques de culture.

La viabilité est calculée en fonction de la surface des explants qui diffèrent entre les cellules de l'invention (4,52cm²) et les explants des inserts dans les plaques de culture (4,16 cm²).

Le dosage MTT démontre que les peaux montées sur le dispositif selon l'invention réagissent très bien, aucune nécrose au niveau des joints n'a été détectée. Il apparait même que la viabilité des explants de peaux est supérieure avec l'utilisation du dispositif selon l'invention en comparaison des inserts en plaque de culture. De meilleurs résultats sont obtenus en termes de survie avec le dispositif objet de l'invention en comparaison des inserts sur plaques de culture. D'après cette mesure l'invention permet d'améliorer le maintien en survie des explants de peau.

### Résultats dosage LDH :

Les résultats sont résumés dans le tableau 2 ci-dessous

| mU/ml | Statique | Invention |
|---|---|---|
| Moyenne | 614,31 | 225,36 |
| SEM | 92,37 | 62,68 |

La quantité de lactate déshydrogénase (LDH) retrouvée dans les milieux de culture est inférieure lors de la mise en œuvre du dispositif objet de l'invention en comparaison des inserts en plaque de culture. Les résultats ont été normalisés par rapport au volume de milieu de culture dans les deux dispositifs ainsi que la surface de l'explant.

Cette étude montre qu'il n'y a pas plus de cytotoxicité avec le dispositif objet de l'invention en comparaison avec les inserts en plaque de culture. Il semble que les explants de peaux soient moins en souffrance lorsqu'elles sont placées dans le dispositif selon l'invention.

Ces résultats corrèlent avec les résultats obtenus pour le dosage du MTT montrant que l'invention permet de mieux maintenir en survie les explants cutanés.

### Exemple 3 : test de fuite

Les inventeurs ont conduit des tests afin d'évaluer le dispositif selon l'invention et notamment rechercher la présence de fuites lors d'expérimentation.

Les études ont été menées sur 3 donneurs en n=4 par donneur.

La méthodologie est la même que celle décrite dans l'exemple 1. Les explants de peau d'oreille de porc ont été congelées pour des raisons de praticité, cela n'ayant pas d'impact pour les études de passage percutané. L'utilisation de peau congelée et non maintenue en survie est autorisée par les recommandations pour l'évaluation du passage percutané (OECD et SCCS).

Afin de faciliter le dosage dans les différents compartiments du modèle, de la caféine marquée au carbone 14 (650 000 dpm/dépôt) est utilisée pour étudier le passage percutané dans le dispositif selon l'invention.

Une solution de caféine à 1% dans un mélange PBS/EtOH (95/5 ; v/v) contenant de la caféine radiomarquée (C14) a été préparée. 10µl/cm² de cette solution est appliqué à la surface de l'explant de peau (application topique). L'étude est menée pendant 24 heures.

A la fin de l'étude, le dispositif est soigneusement démonté pour quantifier la caféine radiomarquée dans chaque compartiment et pièce du dispositif par comptage de la radioactivité par scintillation liquide.

Surface : la surface de la peau est lavée trois fois avec des cotons-tiges imbibés d'un solvant approprié, soit un mélange eau/éthanol 50/50 puis séchée avec un coton-tige. Les cotons-tiges sont placés dans un flacon à scintillation avec 10mL de solvant puis passés aux ultrasons pendant 20 minutes. Un aliquot de 1mL est compté après ajout de 12mL de liquide scintillant par comptage par scintillation liquide.

Peau : la peau est placée dans des flacons en verre contenant 3mL de solvable et les échantillons sont placés 24h à l'étude à 60°C pour permettre une digestion totale des explants de peau. Puis 12mL de liquide scintillant est ajouté et l'échantillon est compté par scintillation liquide.

Milieu récepteur : les milieux sont directement récupérés pour comptage par scintillation liquide après ajout de 12mL de liquide scintillant.

Filtres : les filtres sont placés dans 10mL de solvant (eau/éthanol 50/50) puis passés aux ultrasons pendant 20 minutes. Un aliquot de 1mL est compté après ajout de 12mL de liquide scintillant par comptage par scintillation liquide.

Rinçage du compartiment donneur : le chapeau est lavé à l'aide d'un tampon salivaire imbibé de solvant (eau/éthanol 50/50). Les tampons salivaires sont placés dans un flacon à scintillation avec 10mL de solvant (eau/éthanol) puis passés aux ultrasons pendant 20 minutes. Un aliquot de 1mL est compté après ajout de 12mL de liquide scintillant pour comptage par scintillation liquide.

Joint compartiment donneur : les joints sont placés dans 10mL du solvant (eau/éthanol 50/50) puis passés aux ultrasons pendant 20 minutes. Un aliquot de 1mL est compté après ajout de 12mL de liquide scintillant pour comptage par scintillation liquide.

Joint compartiment récepteur : les joints sont placés dans 10mL du solvant (eau/éthanol 50/50) puis passés aux ultrasons pendant 20 minutes. Un aliquot de 1mL est compté après ajout de 12mL de liquide scintillant pour comptage par scintillation liquide.

Rinçage du compartiment récepteur : 5mL d'un mélange eau/éthanol 50/50 est introduit dans la cellule. Des va-et-vient sont effectués avec une petite pipette puis les 5mL sont récupérés intégralement dans un flacon à scintillation. Puis est effectué un séchage avec un coton-tige qui est rajouté dans le même flacon. Puis ultrasons pendant 20 minutes. Un aliquot de 1mL est compté après ajout de 15mL de liquide scintillant pour comptage par scintillation liquide.

Rinçages tubes : Circulation d'eau distillée dans les tubes. Comptage du liquide directement au compteur à scintillation liquide après ajout de 15mL de liquide scintillant.

### Résultats

Les résultats ont été exprimés en pourcentage de la dose appliquée et sont résumés dans les tableaux 3 et 4 ci-dessous.

| | **Surface** | **Peau** | **Milieux** | **Filtre** | % **total** |
|---|---|---|---|---|---|
| *Moyenne* | 1,35 | 1,99 | 71,75 | 21,07 | 95,90 |
| *SEM* | 0,41 | 0,25 | 2,15 | 1,79 | 0,82 |

| | | | | | |
|---|---|---|---|---|---|
| Moy : moyenne(n=12) ; | | | | | |

| | **Compartiment donneur** | **Joint haut** | **Joint bas** | **Compartiment donneur** | **Tubes** | % **total** |
|---|---|---|---|---|---|---|
| *Moyenn e* | 0,08 | 1,36 | 0,25 | 0,11 | 0,32 | 2,37 |
| *SEM* | 0,01 | 0,29 | 0,03 | 0,02 | 0,08 | 0,36 |

| | | | | | | |
|---|---|---|---|---|---|---|
| Moy : moyenne (n=12); SEM : écart-type moyen. | | | | | | |

Le total retrouvé est retranscrit dans le tableau 5.

| | |
|---|---|
| | **Total** |
| *Moyenne* | 98,27 |
| *SEM* | 1,18 |

L'ensemble de ces résultats permet de montrer que le rendement de récupération de la caféine est très bon et en accord avec les guidelines de passage percutané aussi bien en cosmétique que pharmaceutique (rendement compris entre 90 et 110% de la dose appliquée). Les pourcentages de caféine retrouvés dans les différents compartiments de la peau sont en accord avec les résultats obtenus dans la littérature. Des pourcentages très faibles de radioactivité sont retrouvés dans les différentes pièces du dispositif, en particulier dans les joints haut et bas ainsi que le compartiment récepteur et le compartiment donneur, ce qui montre qu'il n'y a pas de fuite de la solution radiomarquée durant les 24 heures du test et que le pourcentage de radioactivité retrouvé dans les milieux récepteurs correspond bien au pourcentage de caféine ayant diffusée à travers la peau. Ce test permet de montrer que le serrage exercé avec ce dispositif permet une étanchéité suffisante des compartiments donneur et récepteur.

### Exemple 4 : Maintien des capacités métaboliques de la peau

La fonctionnalité des enzymes cutanées a été évaluée pour le dispositif de l'invention en comparaison avec les inserts dans des plaques de culture. Trois molécules métabolisées par des classes d'enzymes cutanés différentes ont été sélectionnées.

La première molécule sélectionnée est le résorcinol qui est métabolisé par la peau directement par des enzymes de phase II, en particulier les UDP-glucuronyltransférases (UGT)et les sulfotransférases (SULT).

La deuxième molécule sélectionnée est la testostérone. Les hydroxylations de la testostérone sont caractéristiques de différentes familles de cytochromes CYP P450 (enzymes de phase I) comme les CYP 2A1, 2B1, 2C11, 2C18, 2C19, 2D9, 3A4 et 3A5 qui biotransforment la testostérone en testostérone monohydroxylée en différentes positions.

La troisième molécule sélectionnée est le propylparaben afin d'évaluer la fonctionnalité des carboxyl esterases, enzymes prépondérantes au niveau de la peau et responsable de la dégradation d'un grand nombre de composés dont la majorité des pro-drogues appliquées par voie topique.

Les études ont été menées sur 3 donneurs en n=2 par donneur.

La méthodologie est la même que celle décrite dans l'exemple 1.

Afin de pouvoir quantifier les pourcentages de composées ayant été métabolisés par les enzymes cutanées et ainsi les quantifier dans les différents compartiments de la peau, des composés radiomarqués au carbone 14 ont été utilisés.

Les analyses ont été effectuées par comptage à scintillation liquide et radio-HPLC. Les explants de peau et les milieux de culture ont été extraits puis analysés par radio-HPLC afin de quantifier les différents métabolites formés.

Ces données ont permis de comparer le passage percutané des composés dans le dispositif de l'invention et les inserts de plaque de culture mais également d'évaluer les capacités de métabolisation des explants cutanés avec le dispositif de l'invention et les inserts des plaques de culture. Ainsi avec l'ensemble de ces tests, les inventeurs démontrent clairement les avantages d'un tel dispositif selon l'invention

Les inventeurs ont évalué les capacités métaboliques d'explants de peaux utilisées soit dans le dispositif selon l'invention soit dans des inserts en plaque de culture, afin de comparer les deux dispositifs, les inserts en plaque de culture étant le modèle de référence.

L'étude a été réalisée sur 6 donneurs en duplicate pour les cellules du dispositif selon l'invention est réalisée, et en parallèle pour les inserts en plaque de culture dans un incubateur classique qui va servir de comparateur. Il s'agit d'une plaque comportant 6 puits d'un diamètre de 35 mm dans lesquels sont placés les inserts en plaque de culture où sont posés les explants de peau. Le système est constitué d'un compartiment récepteur, sous-jacent à la peau, et d'un compartiment donneur en contact avec l'air atmosphérique. Les explants de peau situés à l'interface air-liquide, reposent sur des inserts de culture équipés d'une membrane de 25 mm de diamètre (23 mm de diamètre d'exposition, 4,16 cm²).

L'étude est réalisée sur des explants de peau d'oreille de porc maintenue en survie. Le milieu complet comprend 100 mL de DMEM (Dulbecco's Modified Eagle Medium), sans rouge de phénol, 4 mL de L-glutamine (200 mM), 2 mL de streptomycine/pénicilline (100 µg/mL), 1 mL de fongizone (2,5 µg/mL) et 100 µL de gentamycine (50 µg/mL), 4 mL d'acides aminés essentiels, 2 mL d'acides aminés non-essentiels et 2 mL de sodium pyruvate.

L'étude de métabolisme cutané est réalisée en conditions de dose finie pendant une période de 24 heures et après application unique de 10 nanomoles de substances test dans 40 µL.

Les substances test appliquées à la surface d'un échantillon de peau sont radiomarquées soit un dépôt de 650 000 dpm.

L'expérience est effectuée le jour de réception de la peau pour le maintien de l'activité des enzymes cutanées. La peau est découpée longitudinalement à une épaisseur d'environ 450 ± 50 µm à l'aide d'un dermatome électrique.

Des échantillons de peau sont découpés à l'aide d'un punch 32 mm pour le dispositif et 28 mm pour les plaques à inserts. L'échantillon de peau ainsi prélevé comprend l'ensemble de l'épiderme (environ 20 à 40 µm d'épaisseur) et une partie du derme.

Les explants de peau sont placés sur les inserts dans les plaques de culture ou sur le dispositif. Après une durée de stabilisation de 1 heure dans l'incubateur, les dépôts peuvent être réalisés (10 µL/cm²).

En fin d'expérimentation (24 heures après le dépôt), la radioactivité a été dosée dans les différents compartiments des cellules.

Les différents compartiments du dispositif sont traités comme suit :

Surface : la surface est lavée trois fois avec des cotons tiges imbibés du solvant approprié puis séchée avec un coton tige. Les cotons tiges sont extraits et un aliquot de 1 mL est compté au compteur à scintillation liquide après ajout de 12 mL de liquide scintillant.

Joint cellules haut : les joints sont extraits et un aliquot de 1 mL est compté au compteur à scintillation liquide après ajout de 12 mL de liquide scintillant.

La peau : un réplicat de peau par donneur est placé dans un flacon en verre contenant 3 mL de solvant et placé 24 heures à l'étuve à 60°C pour digestion puis comptage par scintillation liquide. Le second réplicat est découpé en morceaux et extrait. Une centrifugation de 10 minutes à 12 000 rpm à 4°C permet d'obtenir l'extrait contenant le composé parent et les potentiels métabolites. Cet extrait est compté et analysé par radio-HPLC. Les culots sont digérés et la radioactivité est quantifiée par scintillation liquide.

*Stratum corneum :* les peaux sont retirées des cellules et épinglées sur un champ stérile. Un arrachage du *stratum corneum* est effectué à l'aide d'adhésifs (D-squames^{®}). Le premier adhésif est placé seul dans un flacon à scintillation en verre (il sera comptabilisé avec le compartiment « surface »). Les adhésifs sont ensuite poolés deux à deux jusqu'au 15^{ème} adhésif. Les échantillons sont placés une nuit à l'étuve à 60°C après ajout de 3 mL de solvant puis comptés par scintillation liquide.

Filtre : le filtre est extrait et un aliquot de 1 mL est compté au compteur à scintillation liquide après ajout de 12 mL de liquide scintillant.

Joint cellules bas : les joints sont extraits et un aliquot de 1 mL est compté au compteur à scintillation liquide après ajout de 12 mL de liquide scintillant.

Milieu récepteur : les milieux de culture sont collectés dans les tubes en verre ambré de 5 mL. Les volumes des milieux de culture sont déterminés par pesée. Un aliquot de 500 µL est prélevé pour chaque milieu et compté au compteur à scintillation liquide. Le milieu de culture est prélevé puis évaporé sous azote avant analyse en radio-HPLC afin de quantifier les potentiels métabolites formés.

Cupule : le milieu qui est au fond de la cellule est récupéré dans le dernier tube en verre de milieu.

Rinçage chapeau (compartiment donneur) : le chapeau est lavé avec un tampon salivaire imbibé du solvant approprié. Le tampon salivaire est extrait et un aliquot de 1 mL est compté au compteur à scintillation liquide après ajout de 12 mL de liquide scintillant.

Rinçage cellule (compartiment récepteur) : Le compartiment récepteur est rincé avec du solvant puis séché à l'aide d'un coton tige. Après extraction un aliquot de 1 mL est compté au compteur à scintillation liquide après ajout de 12 mL de liquide scintillant.

Les différents compartiments des inserts en plaque de culture sont traités comme suit : la surface de la peau est lavée 3 fois avec des cotons tiges imbibés du solvant approprié puis séchée avec un coton tige. Les coton tiges sont extraits et un aliquot de 1 mL est compté au compteur à scintillation liquide après ajout de 12 mL de liquide scintillant. Les inserts sont extraits et un aliquot de 1 mL est compté au compteur à scintillation liquide après ajout de 12 mL de liquide scintillant. Les cupules sont rincées deux fois avec 1 mL de solvant. Les deux lavages sont collectés dans un flacon à scintillation et comptés au compteur à scintillation liquide après ajout de 12 mL de liquide scintillant. La peau est placée dans des Eppendorfs et conservée à -20°C avant extraction des molécules d'intérêt. Un aliquot (100 µL) de l'extrait est compté après ajout de liquide scintillant et le reste est analysé par radio-HPLC. Pour les milieux récepteurs : un aliquot de 100 µL est effectué pour chaque milieu et compté au compteur à scintillation liquide. L'échantillon est ensuite analysé en radio-HPLC.

### Résultats

Le passage et le métabolisme du résorcinol a été réalisé sur 6 donneurs en duplicate. Les deux systèmes sont démontés et compartimentés 24 heures après application topique du résorcinol. La radioactivité est comptée dans les différents compartiments afin d'évaluer la pénétration du résorcinol à travers la peau. Les milieux récepteurs et les extraits de peaux sont analysés par radio-HPLC afin d'évaluer le métabolisme de la molécule.

La figure 7 montre la pénétration cutanée du résorcinol.

Le pourcentage de la dose appliquée retrouvée à la surface de la peau est identique entre les deux dispositifs (environ 5%). La répartition de la radioactivité entre le *stratum corneum,* la peau et le milieu est différente entre les deux systèmes. Cette différence est liée à l'hygrométrie des conditions expérimentales. En effet, les inserts en plaques de culture sont placés dans un incubateur saturé en eau ce qui intensifie de façon artificielle le passage avec des pourcentages de la dose appliquée surévalués. Ce point est une des problématiques qui ont été résolues avec le dispositif selon l'invention. L'hygrométrie est contrôlée de façon à être plus proche des conditions *in vivo* tout en assurant la viabilité des explants de peau.

### Métabolisme du résorcinol

Le résorcinol et ses métabolites ont été quantifiés par radio-HPLC dans le milieu récepteur et la peau après extraction. Les résultats présentés dans la figure 8 correspondent à la somme en nanomoles de métabolites dans les deux compartiments.

D'après l'état de l'art, le résorcinol est transformé par les enzymes du métabolisme cutané en deux métabolites, un conjugué glucuronide et un conjugué sulfate. Ces deux métabolites sont détectés dans les deux systèmes, dans le dispositif selon l'invention (prototype) et les inserts en plaque de culture. Ceci indique que les voies métaboliques mises en jeu sont identiques entre les deux systèmes : glucuronyl-S-transférases et sulfotransférases et que les enzymes de ces voies sont bien fonctionnelles dans le dispositif selon l'invention.

On observe une différence de proportion de métabolites entre les deux systèmes avec une métabolisation plus importante du résorcinol avec les inserts en plaque de culture. Cette différence est due au fait que ce système est statique. En effet, le résorcinol qui est passé à travers la peau et a atteint le milieu peut être recapté par la peau et ainsi être métabolisé par la peau. Ce « surmétabolisme » est une des limites des inserts sur plaque de culture. Cette problématique a été résolue sur le dispositif de l'invention en introduisant un flux dynamique de milieu sous la peau qui mime la circulation sanguine. Le dispositif selon l'invention est donc plus proche des conditions réelles *in vivo.*

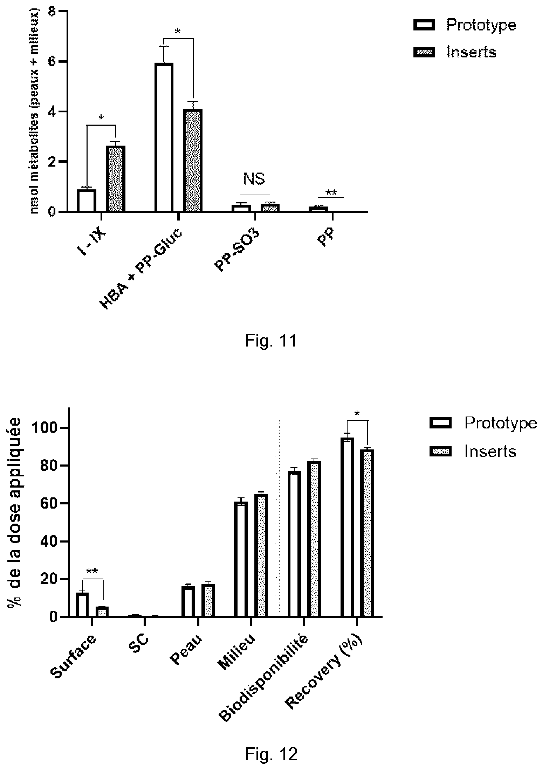

Figures 10 et 11, l'étude de la pénétration cutanée et du métabolisme du propylparaben a été réalisée de la même façon que pour le résorcinol sur 6 donneurs en duplicat. Les deux systèmes, dispositif objet de l'invention (prototype) et inserts en plaque de culture en incubateur classique (inserts), sont démontés et compartimentés 24 heures après application topique de la molécule. La radioactivité est comptée dans les différents compartiments afin d'évaluer la pénétration du propylparaben à travers la peau. Les milieux récepteurs et les extraits de peaux sont analysés par radio-HPLC afin d'évaluer le métabolisme de la molécule.

### Pénétration cutanée du propylparaben

La figure 10 montre la pénétration cutanée du propylparaben. Les résultats représentent la moyenne ± SEM du pourcentage de la dose appliquée mesurée dans chaque compartiment. Les analyses statistiques ont été réalisées avec le logiciel Prism^{®} et correspondent, après avoir vérifié la normalité de la distribution des données au résultat d'un test t. Une différence sera statistiquement significative lorsque p<0,05 (*), p<0,01 (**).

La répartition de la molécule radiomarquée est similaire dans les deux systèmes avec une majorité du pourcentage de la dose appliquée dans les milieux (environ 53%), puis la peau (environ 20%) et la surface (environ 9%). Il n'y a pas de différence statistique entre les deux dispositifs sur ces compartiments. Une différence statistique est observée entre le pourcentage retrouvé dans le *stratum corneum* entre les deux dispositifs néanmoins cette différence statistique ne semble pas biologiquement significative car les coefficients de variations sont élevés, 57% pour le prototype et 41% pour les inserts. De plus, les différences d'hygrométrie entre les deux systèmes peuvent modifier l'hydratation du *stratum corneum,* sachant qu'une hygrométrie de 60% comme le dispositif de l'invention est plus proche des conditions *in vivo.* La biodisponibilité du propylparaben est similaire entre les deux systèmes avec un pourcentage d'environ 75%.

Le contrôle de la température et de l'hygrométrie mis en place pour cette étude a permis d'obtenir des résultats de passage et de biodisponibilité similaires pour cette moléculre entre le dispositif de référence 9inserts) et le dispositif de l'invention (prototype).

### Métabolisme du propylparaben

La figure 11 présente les résultats du métabolisme cutané du propylparaben dans les deux systèmes : dispositif objet de l'invention (prototype) et inserts en plaque de culture en incubateur classique (inserts).

Les résultats représentent la moyenne ± SEM des métabolites ou du propylparaben retrouvés dans la peau et le milieu en nanomoles. Les analyses statistiques ont été réalisées avec le logiciel Prism^{®} et correspondent, après avoir vérifié la normalité de la distribution des données au résultat d'un test t. Une différence sera statistiquement significative lorsque p< 0,05 (*), p<0,01 (**).

Les métabolites détectés dans les deux systèmes sont identiques. Ces résultats sont en adéquation avec l'état de l'art sur le métabolisme du propylparaben.

Des groupes de métabolites ont été formés pour l'analyse pour des contraintes analytiques et pour faciliter la lecture des résultats.

Les métabolites mesurés sont les métabolites I à IX, l'acide hydro-benzoïque (HBA) avec le propylparaben-glucuronide (PP-Gluc) et le propylparaben sulfate (PP-SO3). Le composé parent, le propylparaben, est noté PP. Ces résultats indiquent que, comme pour le résorcinol, les voies métaboliques mises en jeu sont identiques entre les deux systèmes : estérases, glucuronyl-S-transférases et sulfotransférases et que les enzymes de ces voies sont bien fonctionnelles dans le dispositif selon l'invention.

A l'instar du résorcinol, on observe une différence de proportion de métabolites entre les deux systèmes ave une métabolisation plus importante du propylparaben avec les inserts en plaque de culture.

En effet, la quantité de composé parent quantifiée dans le dispositif de l'invention est plus importante que dans le système de référence soit les inserts en plaque de culture. On observe donc à nouveau cette différence due au fait que ce système est statique et qu'il y a un métabolisme supplémentaire du propylparaben dans le système des inserts.

Cette problématique a été résolue sur le dispositif de l'invention en introduisant un flux dynamique de milieu sous la peau qui mime la circulation sanguine. Le dispositif selon l'invention est donc plus proche des conditions réelles *in vivo.*

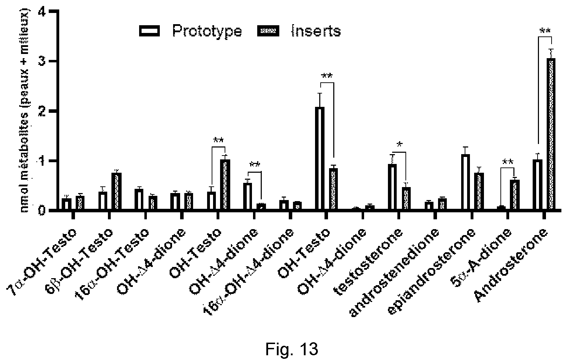

Figures 12 et 13, l'étude de la pénétration cutanée et du métabolisme de la testostérone a été réalisée de la même façon sur 6 donneurs en duplicat pour chaque système. Un ajout de 4% de BSA dans le milieu de culture a été réalisé afin d'améliorer la solubilité de la testostérone et de ses métabolites dans le milieu de culture. Les deux systèmes, dispositif objet de l'invention (prototype) et inserts en plaque de culture en incubateur classique (inserts), sont démontés et compartimentés 24 heures après application topique de la molécule. La radioactivité est comptée dans les différents compartiments afin d'évaluer la pénétration de la testostérone à travers la peau. Les milieux récepteurs et les extraits de peaux sont analysés par radio-HPLC afin d'évaluer le métabolisme de la molécule.

Pénétration cutanée de la testostérone.

La figure 12 montre la pénétration cutanée de la testostérone dans les deux systèmes : dispositif objet de l'invention (prototype) et inserts en plaque de culture en incubateur classique (inserts).

Les résultats représentent la moyenne ± SEM du pourcentage de la dose appliquée mesurée dans chaque compartiment. Les analyses statistiques ont été réalisées avec le logiciel Prism^{®} et correspondent, après avoir vérifié la normalité de la distribution des données au résultat d'un test t. Une différence sera statistiquement significative lorsque p<0,05 (*), p<0,01 (**).

La répartition de la molécule radiomarquée est comparable dans les deux systèmes avec une majorité du pourcentage de la dose appliquée dans les milieux (entre 61 et 65%), puis la peau (environ 16%).

Les quantités retrouvées au niveau du *stratum corneum* sont plus faibles autour de 1% de la dose appliquée. Il n'y a pas de différence statistique entre les deux systèmes sur ces compartiments.

Une différence statistique est observée concernant le pourcentage retrouvé au niveau de la surface de la peau entre les deux systèmes avec un pourcentage plus important sur le dispositif de l'invention. On retrouve cette différence au niveau des rendements de récupération de la molécule (recovery).

En effet, ceux-ci sont plus importants pour le dispositif de l'invention face au dispositif de référence (inserts) ce qui traduit un rendement d'extraction de la surface plus faible pour le dispositif de référence.

Cette différence est donc peu significative car liée à une différence de protocole.

L'aspect important est que la biodisponibilité de la testostérone est similaire entre les deux systèmes avec un pourcentage d'environ 80%. Ce paramètre est le paramètre clé, utilisé à la suite d'expérimentations de passage transcutané, pour les évaluations toxicologiques des molécules.

Comme précédemment pour les résultats obtenus avec le propylparaben, le contrôle de la température, de l'hygrométrie mis en place a permis d'obtenir des résultats de passage et de biodisponibilité similaires pour cette molécule.

Métabolisme de la testostérone.

La figure 13 présente les résultats du métabolisme cutané de la testostérone dans les deux systèmes : dispositif objet de l'invention (prototype) et inserts en plaque de culture en incubateur classique (inserts).

Les résultats représentent la moyenne ± SEM des métabolites ou de la testostérone retrouvés dans la peau et le milieu en nanomoles. Les analyses statistiques ont été réalisées avec le logiciel Prism^{®} et correspondent, après avoir vérifié la normalité de la distribution des données au résultat d'un test t. Une différence sera statistiquement significative lorsque p<0,05 (*), p<0,01 (**).

Les métabolites détectés dans les deux systèmes sont identiques ce qui indique que les voies métaboliques mises en jeu dans les deux systèmes sont similaires.

Ces résultats sont en adéquation avec l'état de l'art et les études antérieures sur le métabolisme de la testostérone.

Les CYP450 sont les enzymes qui interviennent dans le métabolisme de la testostérone. Elles sont donc fonctionnelles dans le dispositif de l'invention.

Il est intéressant de noter que ces enzymes du métabolisme sont particulièrement sensibles et sont rapidement dégradées dans le cadre d'une mauvaise viabilité de la peau.

Les résultats sur le métabolisme de la testostérone sont similaires à ceux obtenus pour le résorcinol et le propylparaben. Une différence de proportion de métabolites entre les deux systèmes avec une métabolisation plus importante du composé parent est observée avec les inserts en plaque de culture.

En effet, la quantité de composé parent quantifiée dans le dispositif de l'invention est plus importante (11,58% de testostérone) que dans le système de référence soit les inserts en plaque de culture (5,11% de testostérone).

Comme expliqué précédemment, cette différence était attendue avec un « surmétabolisme » dans le système des inserts dû à l'aspect statique de ce système qui ne correspond pas à des conditions réelles *in vivo.*

C'est dans ce souci d'être au plus proche des conditions *in vivo* qu'un flux dynamique de milieu sous la peau mimant la circulation sanguine a été mis en place dans le dispositif de l'invention.

La description ci-avant et les exemples de réalisation montrent que l'invention atteint le but visé. En particulier, la conception de la cellule et plus précisément des moyens de serrage de l'explant permettent à la fois d'assurer l'étanchéité du dispositif, évitant la perte de produit ou les fuites, tout en contrôlant la pression sur l'explant afin d'éviter les phénomènes de nécrose.

## Revendications

1. Cellule de diffusion pour l'étude de la pénétration percutanée d'une substance et sa métabolisation sur un explant (250) de peau humaine/animale ou épiderme/peau reconstruit, comprenant :
- ledit explant (250) de peau humaine/animale ou épiderme/peau reconstruit ;
- un puits (220) comprenant une cavité (320) constituant un compartiment récepteur ;
- un chapeau (210) comprenant un compartiment (310) donneur ;
- des moyens de connexion du puits et du chapeau ;
**caractérisée en ce que** l'explant (250) est maintenu entre le compartiment donneur et le compartiment récepteur entre deux joints (271, 272), l'un des joints (272) étant lié au puits dans un logement pratiqué dans ledit puits et l'autre (271) au chapeau dans un logement pratiqué dans ledit chapeau, et qu'il comprend des moyens (211, 221) pour régler la pression exercée par les joints (271, 272) sur l'explant (250) pour assurer l'étanchéité sans entrainer de nécrose de l'explant.

2. Cellule de diffusion selon la revendication 1, dans laquelle les moyens de réglage de la pression exercée par les joints (271, 272) comprennent des moyens d'interface répartis entre le chapeau et le puits et qui définissent, lorsqu'ils sont en contact, une surface de pose du chapeau sur le puits, et des moyens de réglage (221) permettant de modifier la distance entre la surface de pose et le logement de joint pratiqué dans le chapeau ou le logement de joint pratiqué dans le puits.

3. Cellule selon la revendication 2, dans laquelle les moyens d'interface du chapeau et du puits sont maintenus l'un contre l'autre par une force d'attraction magnétique.

4. Cellule selon la revendication 1, comprenant une membrane (350) perméable ou semi-perméable maintenue contre l'explant et apte à maintenir l'explant dans une configuration sensiblement plane.

5. Cellule selon la revendication 3, comprenant des conduits (371, 372) pour le passage d'un fluide dans la cavité du puits, lesquels conduits débouchent dans la cavité (320) du puits, et des moyens, connectés auxdits conduits, pour le contrôle du débit du fluide dans ladite cavité

6. Cellule selon la revendication 5, dans laquelle les moyens de contrôle du débit du fluide dans la cavité (320) comprennent une pompe péristaltique agissant en refoulement dans l'un des conduits (371, 372) et une pompe péristaltique agissant en aspiration dans l'autre conduit (371, 372).

7. Cellule selon la revendication 3, dans laquelle la cavité (320) du puits (220) est délimitée à une de ses extrémités par l'explant (250) et à son extrémité opposée par une plaque de fermeture (325), ladite plaque de fermeture étant constituée d'une matière transparente de sorte à permettre l'observation visuelle de la circulation du fluide dans ladite cavité.

8. Dispositif comprenant une pluralité de cellules selon la revendication 5, et comprenant :
- une enceinte (400) et des moyens de régulation aptes à maintenir la pluralité de cellules dans des conditions de température et d'environnement contrôlée ;
- des moyens (471, 472) pour le passage d'un fluide dans les cavités de puits de chaque cellule ;
- des moyens (490) pour la collecte du fluide passé dans les cavités de puits de chaque cellule.

9. Dispositif selon la revendication 8, comprenant une pluralité de cellules selon la revendication 7 montées dans un rack (640) ledit rack comprenant une surface miroir (641) permettant de refléter l'image observée dans chaque cellule à travers sa plaque de fermeture (325).

10. Dispositif selon la revendication 8, dans lequel les moyens de passage d'un fluide comprennent un réservoir (470) de fluide, une première pompe (471) aspirant le fluide dans ledit réservoir et l'injectant dans la cavité de puits de chaque cellule par l'un des conduits (371) desdites cellules et une deuxième pompe (472) aspirant le fluide dans la cavité de puits de chaque cellule par l'autre conduit (372) desdites cellules et le refoulant dans les moyens de collecte (490).

11. Dispositif selon la revendication 10, dans lequel les moyens de collecte comprennent une station d'entrée (570) comprenant des moyens pour connecter les conduits (572) au refoulement de la deuxième pompe (472), une station de prélèvement (575) comprenant une pluralité becs de remplissage (573) chacun d'entre eux en connexion hydraulique avec un des conduits de refoulement de la deuxième pompe, un rack (520) apte à contenir une pluralité de flacons (530) et à positionner lesdits flacons en vis-à-vis de chaque bec de remplissage (573), des moyens de déplacement (540) relatif dudit rack par rapport à la station de prélèvement (575) de sorte à changer les flacons (530) placé en vis-à-vis de chaque bec de remplissage.

12. Dispositif selon la revendication 11, dans lequel les moyens de collecte comprennent des moyens (990) pour réfrigérer les flacons compris dans le rack (520).

## Patentansprüche

1. Diffusionszelle zur Untersuchung der perkutanen Penetration einer Substanz und ihrer Metabolisierung auf einem Explantat (250) aus menschlicher/tierischer Haut oder rekonstruierter Epidermis/Haut, umfassend:
- das Explantat (250) aus menschlicher/tierischer Haut oder rekonstruierter Epidermis/Haut;
- eine Vertiefung (220), die einen Hohlraum (320) umfasst, der eine Aufnahmekammer bildet;
- eine Kappe (210), die eine Spenderkammer (310) umfasst;
- Mittel zum Verbinden der Vertiefung und der Kappe;
**dadurch gekennzeichnet, dass** das Explantat (250) zwischen der Spenderkammer und der Aufnahmekammer zwischen zwei Dichtungen (271, 272) gehalten wird, wobei eine der Dichtungen (272) mit der Vertiefung in einer in der Vertiefung ausgebildeten Aussparung und die andere (271) mit der Kappe in einer in der Kappe ausgebildeten Aussparung verbunden ist, und dass es Mittel (211, 221) umfasst, um den Druck einzustellen, der von den Dichtungen (271, 272) auf das Explantat (250) ausgeübt wird, um die Abdichtung zu gewährleisten, ohne eine Nekrose des Explantats zu verursachen.

2. Diffusionszelle nach Anspruch 1, wobei die Mittel zum Einstellen des von den Dichtungen (271, 272) ausgeübten Drucks Schnittstellenmittel, die zwischen der Kappe und der Vertiefung verteilt sind und die, wenn sie in Kontakt sind, eine Auflagefläche der Kappe auf der Vertiefung definieren, und Einstellmittel (221) umfassen, die das Ändern des Abstands zwischen der Auflagefläche und der in der Kappe ausgebildeten Aussparung oder der in der Vertiefung ausgebildeten Aussparung ermöglichen.

3. Zelle nach Anspruch 2, wobei die Schnittstellenmittel der Kappe und der Vertiefung durch eine magnetische Anziehungskraft gegeneinander gehalten werden.

4. Zelle nach Anspruch 1, umfassend eine durchlässige oder halbdurchlässige Membran (350), die gegen das Explantat gehalten wird und in der Lage ist, das Explantat in einer im Wesentlichen ebenen Konfiguration zu halten.

5. Zelle nach Anspruch 3, umfassend Kanäle (371, 372) für den Durchlass eines Fluids in den Hohlraum der Vertiefung, wobei die Kanäle in den Hohlraum (320) der Vertiefung münden, und Mittel, die mit den Kanälen verbunden sind, zur Steuerung des Durchflusses des Fluids in den Hohlraum.

6. Zelle nach Anspruch 5, wobei die Mittel zur Steuerung des Durchflusses des Fluids in den Hohlraum (320) eine peristaltische Pumpe umfassen, die in einem der Kanäle (371, 372) als Druckpumpe wirkt, und eine peristaltische Pumpe, die in dem anderen Kanal (371, 372) als Saugpumpe wirkt.

7. Zelle nach Anspruch 3, wobei der Hohlraum (320) der Vertiefung (220) an einem seiner Enden durch das Explantat (250) und an seinem gegenüberliegenden Ende durch eine Verschlussplatte (325) begrenzt ist, wobei die Verschlussplatte aus einem transparenten Material besteht, so dass die visuelle Beobachtung der Zirkulation des Fluids in dem Hohlraum ermöglicht wird.

8. Vorrichtung, umfassend eine Vielzahl von Zellen nach Anspruch 5, und umfassend:
- ein Gehäuse (400) und Regelungsmittel, die geeignet sind, die Vielzahl von Zellen unter kontrollierten Temperatur- und Umgebungsbedingungen zu halten;
- Mittel (471, 472) für den Durchlass eines Fluids in die Hohlräume der Vertiefungen jeder Zelle;
- Mittel (490) zum Auffangen des Fluids, das in die Hohlräume der Vertiefungen jeder Zelle durchgelassen wurde.

9. Vorrichtung nach Anspruch 8, umfassend eine Vielzahl von Zellen nach Anspruch 7, die in einem Gestell (640) montiert sind, wobei das Gestell eine Spiegelfläche (641) umfasst, die es ermöglicht, das in jeder Zelle beobachtete Bild durch ihre Verschlussplatte (325) zu reflektieren.

10. Vorrichtung nach Anspruch 8, wobei die Mittel zum Durchlassen eines Fluids einen Fluidbehälter (470), eine erste Pumpe (471), die das Fluid aus dem Behälter ansaugt und es durch einen der Kanäle (371) der Zellen in den Vertiefungshohlraum jeder Zelle injiziert, und eine zweite Pumpe (472) umfassen, die das Fluid in den Vertiefungshohlraum jeder Zelle durch den anderen Kanal (372) der Zellen ansaugt und es in die Sammelmittel (490) fördert.

11. Vorrichtung nach Anspruch 10, wobei die Auffangmittel eine Eingangsstation (570), die Mittel zum Verbinden der Kanäle (572) mit dem Auslass der zweiten Pumpe (472) umfasst, eine Entnahmestation (575), die eine Vielzahl von Füllstutzen (573) umfasst, die jeweils in hydraulischer Verbindung mit einem der Förderkanäle der zweiten Pumpe stehen, ein Gestell (520), das geeignet ist, eine Vielzahl von Fläschchen (530) aufzunehmen und die Fläschchen gegenüber jedem Füllstutzen (573) zu positionieren, Mittel (540) zum relativen Verschieben des Gestells in Bezug auf die Entnahmestation (575), so dass die gegenüber jedem Füllstutzen platzierten Fläschchen (530) gewechselt werden, umfassen.

12. Vorrichtung nach Anspruch 11, wobei das Sammelmittel Mittel (990) zum Kühlen der im Gestell (520) enthaltenen Fläschchen umfasst.

## Claims

1. Diffusion cell for the study of the percutaneous penetration of a substance and its metabolism on an explant (250) of human/animal skin or reconstructed epidermis/ skin, comprising:
- said explant (250) of human/animal skin or reconstructed epidermis/skin;
- a well (220) comprising a cavity (320) constituting a receiving compartment;
- a cap (210) comprising a donor compartment (310);
- means for connecting the well and the cap;
**characterized in that** the explant (250) is held between the donor compartment and the receiving compartment between two seals (271, 272), one of the seals (272) being connected to the well in a recess formed in said well and the other (271) to the cap in a recess formed in said cap, and **in that** it comprises means (211, 221) for adjusting the pressure exerted by the seals (271, 272) on the explant (250) to ensure sealing without causing necrosis of the explant.

2. Diffusion cell according to claim 1, wherein the means for adjusting the pressure exerted by the seals (271, 272) comprise interface means distributed between the cap and the well and which define, when in contact, a surface for placing the cap on the well, and adjustment means (221) for modifying the distance between the surface for placing the cap and the seal recess formed in the cap or the seal recess formed in the well.

3. Cell according to claim 2, wherein the interface means of the cap and the well are held against each other by a magnetic attraction force.

4. Cell according to claim 1, comprising a permeable or semi-permeable membrane (350) held against the explant and capable of maintaining the explant in a substantially flat configuration.

5. Cell according to claim 3, comprising conduits (371, 372) for passing a fluid into the cavity of the well, which conduits open into the cavity (320) of the well, and means, connected to said conduits, for controlling the flow of the fluid in said cavity.

6. Cell according to claim 5, wherein the means for controlling the flow of the fluid in the cavity (320) comprise a peristaltic pump acting in discharge in one of the conduits (371, 372) and a peristaltic pump acting in suction in the other conduit (371, 372).

7. Cell according to claim 3, wherein the cavity (320) of the well (220) is delimited at one of its ends by the explant (250) and at its opposite end by a closing plate (325), said closing plate being made of a transparent material so as to allow visual observation of the flow of the fluid in said cavity.

8. Device comprising a plurality of cells according to claim 5, and comprising:
- an enclosure (400) and regulation means capable of maintaining the plurality of cells under controlled temperature and environmental conditions;
- means (471, 472) for passing a fluid into the well cavities of each cell;
- collection means (490) for collecting the fluid passed into the well cavities of each cell.

9. Device according to claim 8, comprising a plurality of cells according to claim 7 mounted in a rack (640), said rack comprising a mirror surface (641) making it possible to reflect the image observed in each cell through its closing plate (325).

10. Device according to claim 8, wherein the means for passing a fluid comprise a reservoir (470) of fluid, a first pump (471) sucking the fluid into said reservoir and injecting it into the well cavity of each cell via one of the conduits (371) of said cells and a second pump (472) sucking the fluid into the well cavity of each cell via the other conduit (372) of said cells and delivering it into the collection means (490).

11. Device according to claim 10, wherein the collection means comprise an inlet station (570) comprising means for connecting the conduits (572) to the delivery of the second pump (472), a sampling station (575) comprising a plurality of filling spouts (573) each of them in hydraulic connection with one of the delivery conduits of the second pump, a rack (520) capable of containing a plurality of vials (530) and of positioning said vials facing each filling spout (573), means (540) for moving said rack relative to the sampling station (575) so as to change the vials (530) placed facing each filling spout.

12. Device according to claim 11, wherein the collection means comprise means (990) for refrigerating the vials included in the rack (520).
